Europäisches Patentamt

European Patent Office

Office européen des brevets

(19)

(11) **EP 0 991 318 B1**

(12) **EUROPÄISCHE PATENTSCHRIFT**

(45) Veröffentlichungstag und Bekanntmachung des Hinweises auf die Patenterteilung:
**12.11.2003 Patentblatt 2003/46**

(51) Int Cl.7: **A01N 31/16**, A01N 31/04, A23L 3/349, A23L 3/3472, A61K 7/00

(21) Anmeldenummer: **98937529.0**

(22) Anmeldetag: **22.06.1998**

(86) Internationale Anmeldenummer:
**PCT/EP98/03788**

(87) Internationale Veröffentlichungsnummer:
**WO 98/058540 (30.12.1998 Gazette 1998/52)**

(54) **ADDITIV ZUR HALTBARKEITSVERBESSERUNG UND/ODER STABILISIERUNG VON MIKROBIELL VERDERBLICHEN PRODUKTEN**

ADDITIVE FOR IMPROVING THE STORAGE LIFE OF AND/OR STABILISING MICROBIALLY PERISHABLE PRODUCTS

ADDITIF POUR AMELIORER LA CONSERVABILITE ET/OU POUR STABILISER DES PRODUITS PERISSABLES SUR LE PLAN MICROBIEN

(84) Benannte Vertragsstaaten:
**AT BE CH DE DK ES FI FR GB GR IE IT LI LU NL PT SE**
Benannte Erstreckungsstaaten:
**LT LV SI**

(30) Priorität: **23.06.1997 DE 19726429**

(43) Veröffentlichungstag der Anmeldung:
**12.04.2000 Patentblatt 2000/15**

(73) Patentinhaber: **Schür, Jörg-Peter
41844 Wegberg (DE)**

(72) Erfinder: **Schür, Jörg-Peter
41844 Wegberg (DE)**

(74) Vertreter: **Helbing, Jörg, Dr. Dipl.-Chem. et al
Patentanwälte
von Kreisler-Selting-Werner,
Bahnhofsvorplatz 1 (Deichmannhaus)
50667 Köln (DE)**

(56) Entgegenhaltungen:
**EP-A- 0 103 878          WO-A-96/29895
DE-A- 19 617 278        FR-A- 2 228 434
GB-A- 172 993            GB-A- 790 075
US-A- 5 474 774**

• **PATENT ABSTRACTS OF JAPAN vol. 11, no. 353 (C-457), 18. November 1987 & JP 62 126931 A (T. MASATOSHI), 9. Juni 1987**

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft ein Verfahren und ein Additiv sowie dessen Verwendung zur Haltbarkeitsverbesserung und/oder Stabilisierung von mikrobiell verderblichen Produkten durch Zusatz von Additiven.

Industriell bearbeitete Nahrungs- und Futtermittel, Kosmetika, Pharmazeutika und andere für mikrobielle Verderbnis anfällige Produkte müssen eine gewisse, nicht zu kurze Zeit haltbar sein, um nach einem Transport und Vertrieb über die üblichen Wege unverdorben den Verbraucher zu erreichen. Der Verbraucher erwartet darüber hinaus, daß das erworbene Produkt auch nach dem Kauf nicht sofort verdirbt, sondern, je nach Produkt, einige Tage oder Wochen auf Vorrat gehalten werden kann.

Unbehandelt würden die meisten Nahrungs- und Futtermittel innerhalb weniger Tage verderben, da sich Pilze und/oder Bakterien ungehindert, allenfalls durch Kühlung beeinträchtigt, auf einem für sie idealen Nährboden vermehren könnten. Typische Beispiele sind der Verderb von Brot durch Schimmelpilze, z.B. Aspergillus niger, von Fleischprodukten (z.B. Wurst) durch Enterobakterien oder Lactobacillen, die Kontamination von Geflügel durch Salmonellen und vieles andere mehr. Da Pilze einschließlich Hefen bzw. deren Sporen, Grampositive und Gramnegative Bakterien überall vorhanden sind, wo nicht durch besondere, kostspielige und industriell aus ökonomischen Erwägungen nicht anwendbare Maßnahmen ein steriles Umfeld geschaffen wird, müssen geeignete Gegenmaßnahmen getroffen werden.

Herkömmlicherweise werden daher Nahrungs- und Futtermittel, Kosmetika, Pharmazeutika, Farben, Papier und Zellstoffe und andere verderbliche Produkte mit Konservierungsmitteln haltbar gemacht, die laut der Codex Alimentarius Liste der Food und Agriculture Organisation (FAO/WHO Food Standard Programme) in Division 3 Food Additives Preservatives 3.73 als "synthetische Konservierungsmittel" aufgeführt und meist in Form von chemischen Monosubstanzen oder deren Kombinationen eingesetzt werden.

Aus dem Stand der Technik ist eine Vielzahl von Additiven zur Konservierung von verderblichen Produkten bekannt. Hierzu zählen z.B . Additive auf der Basis von Aromastoffen, Alkoholen, organischen Säuren, Aldehyden, phenolischen Stoffen und ätherischen Ölen. Solche Zusammensetzungen sind beispielsweise in der US-Patentschrift 4.446.161, US 49 27 651, WO 94/ 14 414 der GB 172993 und der DE-OS 3138277 sowie in E. Lück (Chemische Lebensmittelkonservierung, Seite 1977, 1986 Springer-Verlag) beschrieben.

Die in der erwähnten Liste aufgeführten Konservierungsmittel sind bakteriostatisch und/oder fungistatisch wirksam und verbessern die Haltbarkeit wesentlich. Sie werden jedoch von vielen Verbrauchern abgelehnt, da ihre Auswirkungen auf die Gesundheit des Verbrauchers nicht bekannt sind, bzw. schädliche Einflüsse, insbesondere bei wiederholter Aufnahme über einen langen Zeitraum, nicht ausgeschlossen werden können. Nachteilig ist auch, daß alle bisher bekannten Verfahren auf der Änderung des pH-Werts oder $a_w$-Werts beruhen.

Nachteilig bei diesen Konservierungsmitteln ist insbesondere, daß sie regelmäßig hohen Konzentrationen dem Nahrungsmittel zugegeben werden. Dadurch gelangen relativ große Mengen dieser Mittel beim Verzehr auch in den menschlichen Körper. Die Folge sind die heute vielfach gehäuft auftretenden Reaktionen in Form allergischer Erkrankungen.

Eine Alternative zur Konservierung durch Zusatz von synthetischen Konservierungsmitteln ist die thermische Inaktivierung von Keimen, z.B. durch Pasteurisieren. Unter Pasteurisieren versteht man eine thermische Behandlung von 30 bis 120 Minuten Einwirkzeit bei 70 bis 85 °C.

Die Pasteurisierung verbessert die Haltbarkeit derart behandelter Produkte erheblich, ist jedoch technisch aufwendig und verbraucht sehr viel Energie. Die Lebensfähigkeit von Sporen wird darüber hinaus oft nicht oder nur sehr unvollständig beeinträchtigt. Eine Pasteurisierung ist außerdem für temperaturempfindliche Produkte nicht anwendbar oder führt zu einem nicht unerheblichen Qualitätsverlust, da spätestens durch das oftmals notwendige zweite Thermisieren (bis zu 85 °C) der "Frischegrad" des pasteurisierten Produktes nachläßt. Außerdem sind gerade wertvolle Bestandteile von Nahrungsmitteln, Kosmetika oder Pharmazeutika, z.B. Vitamine, Aminosäuren und viele pharmazeutische Wirkstoffe, thermolabil, so daß sich eine thermische Behandlung unter den üblichen Pasteurisierungsbedingungen verbietet.

Eine weitere Möglichkeit zur Verbesserung der Haltbarkeit ist es, das von Verderbnis bedrohte Produkt unter Stickstoff oder $CO_2$ luftdicht zu verpacken oder in Vakuumverpackungen bereitzustellen, wie es z.B. bei gemahlenem Kaffee gehandhabt wird. Diese Verfahren sind jedoch teuer und aufwendig und daher für viele Nahrungsmittel nicht anwendbar.

WO96/29895 offenbart ein Verfahren zur Haltbarkeitsverbesserung von mikrobiell verderblichen Produkten, bei denen während der Herstellung, Verarbeitung oder Verpackung der Produkte deren Oberfläche oder Umgebung mit einem Mikrobizid beaufschlagt wird, welches ein oder mehrere GRAS (Generally Recognized AS Safe) Aromastoffe wie Alkohole, Aldehyde, Phenole, Acetate, Säuren, Ester, Terpene, Acetale, Polyphenol und/oder ätherische Öle enthält, sowie das entsprechende Prozess-Hilfsmittel (Mikrobizid) selber.

Aufgabe der vorliegenden Erfindung ist es demgemäß, verbesserte Additive zur Haltbarkeitsverbesserung und/oder Stabilisierung von mikrobiell verderblichen Produkten durch Zusatz von Additiven zur Verfügung zu stellen.

Diese Aufgabe wird gelöst durch ein Additiv enthaltend

(i) ein Gemisch aus

a) Polyphenol und
b) Benzylalkohol und gegebenenfalls weiteren GRAS(Generally Recognized AS Safe)-Aroma-Alkoholen oder

(ii) ein Gemisch aus

c) Benzylalkohol und
d) wenigstens einem weiteren GRAS-Aroma-Alkohol,

wobei das Mischverhältnis der Komponenten a) zu b) und c) zu d) jeweils zwischen 1:1 bis 1:10.000 und 10.000:1 bis 1:1 liegt, und wobei die GRAS-Aromastoffe ausgewählt sind aus Acetoin, Ethanol, 1-Propanol, 2-Propanol, Propylenglykol, Glycerin, n-Butylalkohol, 2-Methyl-1-propanol, Hexanol, L-Menthol, Octylalkohol, Zimtalkohol, 1-Phenylethanol, Heptanol, 1-Pentanol, 3-Methyl-1-butanol, 4-Methoxybenzylalkohol, Citronellol, n-Decanol, Geraniol, 3-Hexenol, Dodecanol, Linalool, Nerolidol, Nonadienol, Nonylalkohol, Rhodihol, Terpineol, Borneol, Clineol, Anisol, Cuminylalkohol, 10-Undecen-1-ol und 1-Hexadecanol.

Im folgenden werden die erfindungsgemäß vorzugsweise einsetzbaren Stoffe im einzelnen näher beschrieben:

Als Polyphenol (Komponenten a)) kommen auch deren mögliche Derivate, -salze, -säuren, -ester, -oxidasen, - frei und -verethert, -natürlich in Betracht. Beispiele für einsetzbare Verbindungen sind:

Brenzcatechin, Resorcin, Hydrochinon, Phloroglucin, Ppyrogallol, Hexahydrobenzol, Usninsäure, Acylpolyphenole, Lignine, Anthocyanidine, Flavone, Catechine, Tannine, Gallussäurederivate, Carnosol, Carnosolsäure, (2,5 - Dihydroxyphenyl) Carboxyl und (2,5 - Dihydroxyphenyl) Alkylen Carboxyl, -substitutionen, -derivate, -salze, - ester, -amide, Kaffesäure, -ester, -amide, Gallotannin, Gerbsäuren, Pyrogallol, Gallus-Gelbsäuren, Flavonoide: Flavon, Flavonol, Isoflavon, Gossypetin, Myricetin, Robinetin, Apigenin, Morin, Taxi Folin, Eriodictyol, Naringin, Rutin, Hesperidin, Troxerutin, Chrysin, Tangeritin, Luteolin, Epigallocatechin-Gallat, Quercetin, Fisetin, Kaempferol, Galangin, Rotenoide, Aurone, Flavonole, -diole. Extrakte aus z. B. Camellia, Primula.

Die genannten Polyphenole werden erfindungsgemäß in Kombination mit GRAS-Aroma -Alkoholen eingesetzt. GRAS-Aroma Alkohole sind von der FDA Behörde zur Verwendung in Nahrungsmitteln als gewerbesicher anerkannt (G.R.A.S= Generaly Recognized As Safe In Food). Hierdurch unterscheiden sich die erfindungsgemäßen Alkohole insbesondere von denjenigen, die in der US-4.446.161 eingesetzt werden.

Bei den erwähnten GRAS-Aroma -Alkoholen handelt es sich um solche Alkohole, die in FEMA/FDA GRAS Flavour Substances Lists GRAS 3-15 Nr. 2001-3815 (Stand 1997) genannt sind. In dieser Liste sind natürliche und naturidentische Aromastoffe aufgeführt, die von der amerikanischen Gesundheitsbehörde FDA zur Verwendung in Nahrungsmitteln zugelassen sind (FDA Regulation 21 CFR 172.515 für naturidentische Aromastoffe (Synthetic Flavoring Substances und Adjuvants) und FDA Regulation 21 CFR 182.20 für natürliche Aromastoffe (Natural Flavoring Substances und Adjuvants).

Erfindungsgemäß kann die Komponente a) mit einem oder mehreren GRAS - Aroma-Alkoholen (Komponenten b)) verwendet werden. Bevorzugt wird erfindungsgemäß der Einsatz eines GRAS-Aroma-Alkohols. Besonders bevorzugt ist die Verwendung von zwei GRAS-Aroma-Alkoholen.

Im einzelnen können beispielsweise folgende GRAS-Aroma-Alkohole (Komponente a), c), d)) zum Einsatz kommen:

Benzylalkohol, Acetoin (Acetylmethylcarbinol), Ethylalkohol (Ethanol), Propylalkohol (1-Propanol), iso-Propylalkohol (2-Propanol, Isopropanol), Propylenglykol, Glycerin, n-Butylalkohol (n-Propylcarbinol), isoButylalkohol (2-Methyl-1-propanol), Hexylalkohol (Hexanol), L-Menthol, Octylalkohol (n-Octanol), Zimtalkohol (3-Phenyl-2-propen-1-01), α-Methylbenzylalkohol (1-Phenylethanol), Heptylalkohol (Heptanol), n-Amylalkohol (1-Pentanol), iso-Amylalkohol (3-Methyl-1-butanol), Anisalkohol (4-Methoxybenzylalkohol, p-Anisalkohol), Citronellol, n-Decylalkohol (n-Decanol), Geraniol, β-γ-Hexenol (3-Hexenol), Laurylalkohol (Dodecanol), Linalool, Nerolidol, Nonadienol (2,6-Nonadien-1-ol), Nonylalkohol (Nonanol-1), Rhodinol, Terpineol, Borneol, Clineol (Eucalyptol), Anisol, Cuminylalkohol (Cuminol), 10-Undecen-1-ol, 1-Hexadecanol.

Zu den erfindungsgemäß besonders bevorzugten GRAS-Aroma-Alkoholen gehört Benzylalkohol. Eine demgemäß besonders bevorzugte Kombination enthält Polyphenol (Komponente a)) und Benzylalkohol (Komponente b)).

Dieses Gemisch kann gegebenenfalls weitere GRAS-Aroma-Alkohole enthalten.

Erfindungsgemäß ist es aber auch möglich, daß anstelle des Polyphenols GRAS-Aroma-Alkohole (Komponente d)) vorliegen. D.h., es kann Benzylalkohol (Komponente c)) im Gemisch mit weiteren GRAS-Aroma-Alkoholen (Komponente d)) verwendet werden.

Das Mischungsverhältnis von Polyphenol (Komponente a)) bzw. Benzylalkohol (Komponente c)) zu den GRAS-Aroma-Alkoholen(Komponenten b) und d)) liegt vorzugsweise zwischen 1:1 und 1:10.000 oder 10.000:1 und 1:1, besonders bevorzugt zwischen 1:1.000 und 1:1 oder 1:1 und 1:1.000, ganz besonders bevorzugt zwischen 1:1 bis 1:100 und 100:1 bis 1:1.

In den Komponenten a), b), c) und d) können jedoch zusätzlich verschiedene Alkohole eingesetzt werden. Vorzugsweise handelt es sich dabei um einwertige oder mehrwertige Alkohole mit 2 bis 10 C-Atomen vorzugsweise 2 bis 7 C-Atomen.

Vorzugsweise werden solche Mengen an Polyphenol und Alkoholen eingesetzt, daß das Mischungsverhältnisvon Alkohol zu Polyphenol zwischen 1:1 und 1:1.000 oder Polyphenol zu Alkoholen zwischen 1:1 und 1:1.000, insbesondere 1:1 bis 1:100 und 100:1 bis 1:1 beträgt.

Die wie folgt Beschriebenen Komponenten in dem erfindungsgemäßen Additiv sind Aromastoffe, die in der FEMA/FDA GRAS Flavour Substances Liste als G.R.A.S (Generally Recognized As Safe In Food) 3-15 Nr. 2001-3815 (Stand 1997) anerkannt sind.

Weiterhin sind in dem erfindungsgemäßen Additiv als Komponente e) Säuren und/oder deren pysiologisch verträgliche Salze einsetzbar. Vorzugsweise kommen organische Säure und/oder deren Salze zum Einsatz. Hierbei handelt es sich bevorzugt um solche Verbindungen, die 1 bis 15 C-Atome, vorzugsweise 2 bis 10 C-Atome enthalten.

Im einzelnen können beispielsweise folgende Säuren zum (Komponente e)) Einsatz kommen:

Essigsäure, Aconitsäure, Adipinsäure, Ameisensäure, Apfelsäure (1-Hydroxybernsteinsäure), Capronsäure, Hydrozimtsäure (3-Phenyl-1-propionsäure), Pelagonsäure (Nonansäure), Milchsäure (2-Hydroxypropionsäure), Phenoxyessigsäure (Glykolsäurephenylether), Phenylessigsäure (α-Toluolsäure), Valeriansäure (Pentansäure), isoValeriansäure (3-Methylbutansäure), Zimtsäure (3-Phenylpropensäure), Citronensäure, Mandelsäure (Hydroxyphenylessigsäure) Weinsäure (2,3-Dihydroxybutandisäure; 2,3-Dihydroxybernsteinsäure), Fumarsäure, z.B. Milchsäure, bevorzugt.

Ferner können in dem erfindungsgemäßen Additiv als Komponente f) folgende Verbindungen zum Einsatz kommen:

Als Phenole sind z.B .Thymol, Methyleugenol, Acetyleugenol, Safrol, Eugenol, Isoeugenol, Anethol, Phenol, Methylchavicol (Estragol; 3-4-Methoxyphenyl-1-propen), Carvacrol, α-Bisabolol, Fornesol, Anisol (Methoxybenzol), Propenylguaethol (5-Prophenyl-2-ethoxyphenol) verwendbar.

Als Acetate (Komponente g)) kommen z.B .Iso-Amylacetat (3-Methyl-1-butylacetat), Benzylacetat, Benzylphenylacetat, n-Butylacetat, Cinnamylacetat (3-Phenylpropenylacetat), Citronellylacetat, Ethylacetat (Essigester), Eugenolacetat (Acetyleugenol), Geranylacetat, Hexylacetat (Hexanylethanoat), Hydrocinnamylacetat (3-Phenyl-propylacetat), Linalylacetat, Octylacetat, Phenylethylacetat, Terpinylacetat, Triacetin (Glyceryltriacetat), Kaliumacetat, Natriumacetat, Natriumdiacetat, Calciumacetat zum Einsatz.

Als Ester (Komponente h)) ist z.B .Allicin verwendbar.

Als Terpene (Komponente i)) kommen z.B .Campher, Limonen, β-Caryophyllen in Betracht.

Zu den einsetzbaren Acetalen (Komponente j)) zählen z.B . Acetal, Acetaldehydibutylacetal, Acetaldehyddipropylacetal, Acetaldehydphenethylpropylacetal, Zimtaldehydethylenglycolacetal, Decanaldimethylacetal, Heptanaldimethylacetal, Heptanalglycerylacetal, Benzaldehydpropylenglykolacetal.

Einsetzbar sind auch Aldehyde (Komponente k)), z.B .Acetaldehyd, Anisaldehyd, Benzaldehyd, iso-Butylaldehyd (Methyl-1-propanal), Citral , Citronellal, n-Caprinaldehyd (n-Decanal), Ethylvanillin, Fufurol, Heliotropin (Piperonal), Heptylaldehyd (Heptanal), Hexylaldehyd (Hexanal), 2-Hexenal (β-Propylacrolein), Hydrozimtaldehyd (3-Phenyl-1-propanal), Laurylaldehyd (Dodecanal), Nonylaldehyd (n-Nonanal), Octylaldehyd (n-Octanal), Phenylacetaldehyd (1-Oxo-2-phenylethan), Propionaldehyd (Propanal), Vanillin, Zimtaldehyd (3-Phenylpropenal), Perillaaldehyd, Cuminaldehyd.

Vorzugsweise sind erfindungsgemäß auch Lösungsvermittler (Komponente I)) als Komponente in dem Additiv vorhanden. Denn bei den erfindungsgemäß eingesetzten Additiven handelt es sich im Prinzip um Aromastoffe. Die meisten der in der GRAS FEMA-Liste aufgeführten Aromastoffe sind nicht wasserlöslich, d.h. hydrophob. Werden sie in hauptsächlich fetthaltigen Nahrungsmitteln eingesetzt, so sind sie aufgrund ihres lipophilen Charakters direkt ohne Lösungsmittel verwendbar. Der Anteil lipophiler Nahrungsmittel ist jedoch relativ. gering. Um in den meistens hydrophilen Nahrungs- oder Futtermitteln, Kosmetika oder Pharmazeutika ihre Wirkung entfalten zu können, werden sie bevorzugt in Verbindung mit einem wasserlöslichen Lösungsvermittler eingesetzt. Hierbei handelt es sich vorzugsweise um Glycerin, Propylenglycol, Wasser Speiseöle oder Fette.

Erfindungsgemäß einsetzbar sind beispielsweise auch die im folgenden aufgeführten ätherischen Öle und/ oder alkoholischen, glycolischen oder durch $CO_2$-Hochdruckverfahren erhaltenen Extrakte (Komponente m)) aus den Pflanzen:

a) Öle bzw. Extrakte mit hohem Anteil an Alkoholen:

Melisse, Koriander, Kardamon, Eukalyptus;

b) Öle bzw. Extrakte mit hohem Anteil an Aldehyden:

Eukalyptus citriodora, Zimt, Zitrone, Lemongras, Melisse, Citronella, Limette, Orange;

c) Öle bzw. Extrakte mit hohem Anteil an Phenolen:

Oreganum, Thymian, Rosmarin, Orange, Nelke, Fenchel, Campher, Mandarine, Anis, Cascarille, Estragon und Piment;

d) Öle bzw. Extrakte mit hohem Anteil an Acetaten:

Lavendel;

e) Öle bzw. Ektrakte mit hohem Anteil an Estern:

Senf, Zwiebel, Knoblauch;

f) Öle bzw. Extrakte mit hohem Anteil an Terpenen:

Pfeffer, Pomeranze, Kümmel, Dill, Zitrone, Pfefferminz, Muskatnuß.

Das Mischungsverhältnis der Komponenten a) bzw. c) zu b), d), e), f), g), h), i), j), k), l), m), kann jeweils zwischen 1:1 bis 1:10.000 und 10.000:1 bis 1:1, vorzugsweise zwischen 1:1 bis 1:1000 und 1000:1 bis 1:1 liegen.

Die beschriebenen Additive werden vorzugsweise zur Haltbarkeitsverbesserung und Stabilisierung von folgenden Gruppen von Nahrungsmitteln verwendet:

Brot, Backwaren, Backmittel, Backpulver, Puddingpulver, Getränken, diätetischen Lebensmitteln, Essenzen, Feinkost, Fisch und Fischprodukten, Kartoffeln und Produkten auf Kartoffelgrundlage, Gewürzen, Mehl, Margarine, Obst und Gemüse und Produkten auf Grundlage von Obst und Gemüse, Sauerkonserven, Stärkeprodukten, Süßwaren, Suppen, Teigwaren, Fleisch- und Fleischwaren, Milch-, Molkerei- und Käseprodukten, Geflügel und Geflügelprodukten, Ölen, Fetten und öl- oder fetthaltigen Produkten.

Die erfindungsgemäßen Additive sind insbesondere gegen Schimmelpilze, Hefen und Bakterien (Grampositive und Gramnegative) wirksam. Vor allem gegen pathogene Erreger (Enterobacteriaceaen, z.B. E. Coli, Salmonellen, Enterokokken, z.B . Staphylokokken, Streptokokken, wie auch gegen Verderbniserreger wie z.B. Milchsäurebakterien z.B . Lactobacillus vulgaris, Schimmelpilze, z.B . Aspergillus niger, Hefen, z.B . Endomyces tibuliger, wirken sie hervorragend. Ebenso werden die erfindungsgemäßen Additive und Viren reduzierend gegen mikrobielle Toxine, z. B. Aflatoxine, Enterotoxine.

Die Additive werden vorzugsweise in Mengen von 1 ppm bis 10 Gew.%, vorzugsweise 1 ppm bis 1,0 Gew.% dem mikrobiell verderblichen Produkt zugesetzt. Besonders bevorzugte Mengen sind 0,001 Gew.% bis 0,5 Gew.%. Ganz besonders sind bevorzugt 0,002 Gew.% bis 0,25 Gew.%.

Es ist erfindungsgemäß überraschend, daß die Wirkung der erfindungsgemäßen Additive bereits bei Anwendung der genannten geringen Konzentrationen auftritt. Dies ist um so überraschender, als die mit den erfindungsgemäßen Additiven behandelten Nahrungsmittel eine erheblich längere Haltbarkeit aufweisen als die mit herkömmlichen Konservierungsstoffen behandelten verderblichen Produkte.

Überraschend ist auch, das die beschriebenen Vorteile schon bei mikrobischen Einwirkzeiten von weniger als 24 h, insbesondere als 60 Minuten, vorzugsweise 1 - 60 Minuten, höchst bevorzugt 5 - 15 Minuten auftreten.

Die erfindungsgemäßen Additive führen überraschenderweise zu keinen Nachteilen im Geschmack, Geruch oder Farbe bei dem behandelten Nahrungsmittel. Ein besonderer Vorteil gegenüber dem bisherigen Stand der Technik ist, daß keinerlei Verschiebungen des pH-Werts oder $a_w$-Werts zu verzeichnen sind. D.h., die Wirkung der eingesetzten Additive ist überraschenderweise unabhängig vom pH-Wert und $a_w$-Wert. Ebenso überraschend ist es, daß die Additive unabhängig von der Feuchtigkeit, dem Fett-, Eiweiß- und Kohlenhydratgehalt verwendbar sind. Schließlich sind die erfindungsgemäßen Kombinationen unempfindlich gegen Temperaturschwankungen im Bereich zwischen -30°C und 200°C, d.h. sowohl kälte- als auch hitzeunempfindlich.

Zusätzlich zu den beschriebenen Additiven können vor, während oder nach Abschluß des Prozesses zur Herstellung, Verarbeitung oder Verpackung der Produkte deren Oberflächen und/oder deren Umgebung, insbesondere die Umgebungsluft und/oder die Oberflächen der unmittelbar oder mittelbar mit den Produkten in Kontakt kommenden Geräte oder sonstige Materialien mit einem oder mehreren Prozeßhilfsmitteln beaufschlagt werden, die mindestens zwei Aromastoffe, enthalten. D.h., neben der Verwendung der Additive kann erfindungsgemäß auch eine äußerliche Behandlung mit Prozeßhilfsmitteln erfolgen.

Die in den Prozeßhilfsmitteln enthaltenen Aromastoffe sind ausschließlich Naturstoffe natürliche oder naturidentische Aromastoffe, die gemäß FEMA als sicher (GRAS - Generally Recognized As Safe) anerkannt sind. Bei der erwähnten Liste handelt es sich um FEMA/FDA GRAS Flavour Substances Lists GRAS 3-15 Nr. 2001-3815 (Stand 1997), die natürliche und naturidentische Aromastoffe aufführt, die von der amerikanischen Gesundheitsbehörde FDA zur Verwendung in Nahrungsmitteln zugelassen sind (FDA Regulation 21 CFR 172.515 für naturidentische Aromastoffe (Synthetic Flavoring Substances und Adjuvants) und FDA Regulation 21 CFR 182.20 für natürliche Aromastoffe (Natural Flavoring Substances und Adjuvants). Die diese FDA-Normen erfüllenden Aromastoffe dürfen "quantum satis" eingesetzt werden, d.h. sie dürfen bis zu der Höchstkonzentration im Nahrungsmittel enthalten sein, in der sie noch keine geruchliche oder geschmackliche Beeinträchtigung des Nahrungsmittels, dem sie zugesetzt werden, bewirken. Die gemäß FEMA aufgeführten Aromastoffe decken sich weitgehend mit den in der entsprechenden europäischen Norm COE enthaltenen Stoffen.

Erfindungsgemäß dürfen außerdem die gemäß Artikel V European Community Directive Flavourings (22.06.88) als "NAT4" klassifizierten Aromastoffe verwendet werden, vorausgesetzt, sie gelten gemäß der zuvor erwähnten FEMA GRAS-Liste als sicher. NAT4-Substanzen sind Substanzen, die unter bestimmten Voraussetzungen als naturidentisch zu deklarieren sind, z.B., wenn diese Substanzen in Verbindung und als Bestandteil mit einem natürlichen oder naturidentischen Aromastoff eingesetzt werden.

Der besondere Vorteil der Prozeßhilfsmittel ist, daß es aufgrund seiner in der FEMA GRAS-Liste aufgeführten und von der US-Gesundheitsbehörde FDA, der wohl kritischsten Gesundheitsbehörde überhaupt, als unbedenklich anerkannten Bestandteile im "quantum satis"-Konzentrationsbereich ohne weiteres Nahrungsmitteln zugesetzt werden kann.

Ein weiterer besonderer Vorteil liegt darin, daß die Prozeßhilfsmittel den Geschmack und Geruch der behandelten Produkte nicht beeinflussen.

Die erfindungsgemäßen Prozeßhilfsmittel werden beispielsweise in Form von Schmiermitteln, Emulgier- und Reinigungsmitteln, Sprühmitteln, Vernebelungsmitteln, gasphasenaktiven Mitteln, Wärmeübertragungsmitteln sowie Schneid- oder Trennmitteln eingesetzt. Ebenso können die Prozeßhilfsmittel als Zusätze zu den genannten Mitteln eingesetzt werden.

Es ist wesentlich, daß die Prozeßhilfsmittel nicht den Nahrungsmitteln beigegeben werden bzw. mit diesen vermischt werden. Vielmehr werden nur die Oberflächen bzw. Schnittflächen der Nahrungsmittel mit den Prozeßhilfsmitteln beaufschlagt. Dies kann dadurch geschehen, daß die Nahrungsmitteloberflächen bzw. Schnittflächen direkt mit den Prozeßhilfsmitteln beaufschlagt werden. Ebenso ist es aber auch möglich, die Oberflächen von Geräten, Produktionsmaschinen, Verpackungseinrichtungen, Transporteinrichtungen, Verpackungsmaterialien sowie die Umgebungsluft mit dem Prozeßhilfsmittel zu versetzen.

Es ist überraschend, daß die mikrobizide Wirkung der Prozeßhilfsmittel bereits bei Anwendung geringer Konzentrationen auftritt. Nur 0,01 bis 5 g/kg, vorzugsweise 0,05 bis 2g/kg, besonders bevorzugt 0,05 bis 1g/kg Nahrungsmittel werden bei deren Beaufschlagung verwendet. Bei dem Einsatz für die Umgebungsluft werden nur 0,001 bis 10 g/m$^3$ Luft beispielsweise eingesetzt. Für die Oberflächen von Geräten werden sogar nur 0,000001g bis 0,1 g/cm$^2$ Oberfläche verwendet.

Bei Einhaltung dieser Konzentrationen liegen die in den Nahrungsmitteln nachweisbaren Mengen nur bei 0,001 Gew.-%. Hingegen werden nach dem Stand der Technik regelmäßig 0,1 bis 3 Gew.-% Konservierungsstoff in den Nahrungsmitteln vorhanden sein. Trotz dieser äußerst geringen Konzentrationen ist es erfindungsgemäß überraschend, daß gegenüber herkömmlich konservierten Nahrungsmitteln eine Haltbarkeitsverlängerung von bis zu 50 % erzielt werden kann.

Es ist besonders hervorzuheben und erstaunlich, daß bereits durch Prozeßhilfsmittel die indirekt auf Nahrungsmittel aufgebracht werden, bereits 0,001 Gew.-% ausreichen, um eine Haltbarkeitsstabilisierung bzw. -verbesserung bei erhöhter Produktqualität zu erreichen.

Diese Wirkung ist um so überraschender, als die mikrobizide Wirkungszeit der erfindungsgemäß eingesetzten Aromastoffe unter 24 Stunden, vorzugsweise unter 12 Stunden liegt. Ganz besonders bevorzugt ist es, Prozeßhilfsmittel und Konzentrationen so auszuwählen, daß die mikrobizide Wirkungszeit unter 1 Stunde, vorzugsweise unter 15 Minuten liegt.

Im Gegensatz dazu ist es das Ziel der herkömmlichen Konservierungsstoffe, möglichst lange, d.h. über Wochen und Monate, in dem Lebensmittel wirksam zu sein. Trotz der sehr kurzen Wirkungszeiten der erfindungsgemäß eingesetzten Prozeßhilfsmittel ist die Haltbarkeit gegenüber den nach dem Stand der Technik mit herkömmlichen Konservierungsstoffen bzw. Konservierungsverfahren behandelten Lebensmitteln signifikant erhöht. Erfindungsgemäß ist demgemäß bei Kombination der oben beschriebenen Additive und des Prozeßhilfsmittels es überraschend möglich, mit erheblich geringeren Mengen zu arbeiten, als dies beim Einsatz der bisher nach dem Stand der Technik üblichen Konservierungsstoffe erforderlich war.

Das erfindungsgemäß einsetzbare Prozeßhilfsmittel umfaßt Aromastoffe, die ausgewählt sind aus der Gruppe der Alkohole, Aldehyde, Phenole, Acetate, Säuren, Ester, Terpene, Acetale und deren physiologisch verträglichen Salze, etherischen Ölen und Pflanzenextrakten.

Bevorzugte Ausführungsformen der erfindungsgemäßen Prozeßhilfsmittel umfassen ein oder mehrere Aromastoffe, die aus einer oder mehreren der folgenden Gruppen ausgewählt sind:

I. Alkohole

Acetoin (Acetylmethylcarbinol), Ethylalkohol (Ethanol), Propylalkohol (1-Propanol), iso-Propylalkohol (2-Propanol, Isopropanol), Propyfenglykol, Glycerin, Benzylalkohol, n-Butylalkohol (n-Propylcarbinol), isoButylalkohol (2-Methyl-1-propanol), Hexylalkohol (Hexanol), L-Menthol, Octylalkohol (n-Octanol), Zimtalkohol (3-Phenyl-2-propen-1-01), $\alpha$-Methylbenzylalkohol (1-Phenylethanol), Heptylalkohol (Heptanol), n-Amylalkohol (1-Pentanol), iso-Amylalkohol (3-Methyl-1-butanol), Anisalkohol (4-Methoxybenzylalkohol, p-Anisalkohol), Citronellol, n-Decylalkohol (n-Decanol), Geraniol, $\beta$-$\gamma$-Hexenol (3-Hexenol), Laurylalkohol (Dodecanol), Linalool, Nerolidol, Nonadienol (2.6-Nonadien-1-ol), Nonylalkohol (Nonanol-1), Rhodinol, Terpineol, Borneol, Clineol (Eucalyptol), Anisol, Cuminylalkohol (Cuminol), 10-Undecen-1-ol, 1-Hexadecanol.

II. Aldehyde

Acetaldehyd, Anisaldehyd, Benzaldehyd, iso-Butylaldehyd (Methyl-1-propanal), Citral , Citronellal, n-Caprinaldehyd (n-Decanal), Ethylvanillin, Fufurol, Heliotropin (Piperonal), Heptylaldehyd (Heptanal), Hexylaldehyd (Hexanal), 2-Hexenal ($\beta$-Propylacrolein), Hydrozimtaldehyd (3-Phenyl-1-propanal), Laurylaldehyd (Dodecanal), Nonylaldehyd (n-Nonanal), Octylaldehyd (n-Octanal), Phenylacetaldehyd (1-Oxo-2-phenylethan), Propionaldehyd (Propanal), Vanillin, Zimtaldehyd (3-Phenylpropenal), Perillaaldehyd, Cuminaldehyd.

III. Phenole

Thymol, Methyleugenol, Acetyleugenol, Safrol, Eugenol, Isoeugenol, Anethol, Phenol, Methylchavicol (Estragol; 3-4-Methoxyphenyl-1-propen), Carvacrol, $\alpha$-Bisabolol, Fornesol, Anisol (Methoxybenzol), Propenylguaethol (5-Prophenyl-2-ethoxyphenol).

IV. Acetate

iso-Amylacetat (3-Methyl-1-butylacetat), Benzylacetat, Benzylphenylacetat, n-Butylacetat, Cinnamylacetat (3-Phenylpropenylacetat), Citronellylacetat, Ethylacetat (Essigester), Eugenolacetat (Acetyleugenol), Geranylacetat, Hexylacetat (Hexanylethanoat), Hydrocinnamylacetat (3-Phenyl-propylacetat), Linalylacetat, Octylacetat, Phenylethylacetat, Terpinylacetat, Triacetin (Glyceryltriacetat), Kaliumacetat, Natriumacetat, Natriumdiacetat, Calciumacetat.

V. Säuren und/oder deren pnysiologisch verträgliche Salze

Essigsäure, Aconitsäure, Adipinsäure, Ameisensäure, Apfelsäure (1-Hydroxybernsteinsäure), Capronsäure, Hydrozimtsäure (3-Phenyl-1-propionsäure), Pelagonsäure (Nonansäure), Milchsäure (2-Hydroxypropionsäure), Phenoxyessigsäure (Glykolsäurephenylether), Phenylessigsäure ($\alpha$-Toluolsäure), Valeriansäure (Pentansäure), isoValeriansäure (3-Methylbutansäure), Zimtsäure (3-Phenylpropensäure), Citronensäure, Mandelsäure (Hydroxyphenylessigsäure) Weinsäure (2,3-Dihydroxybutandisäure; 2,3-Dihydroxybernsteinsäure), Fumarsäure, Tanninsäure.

VI. Ester

Allicin.

VII. Terpene

Campher, Limonen, β-Caryophyllen.

VIII. Acetale

Acetal, Acetaldehydibutylacetal, Acetaldehyddipropylacetal, Acetaldehydphenethylpropylacetal, Zimtaldehydethylenglycolacetal, Decanaldimethylacetal, Heptanaldimethylacetal, Heptanalglycerylacetal, Benzaldehydpropylenglykolacetal.

IX. Polyphenol

X. Etherische Öle und/oder alkoholische, glykolische oder durch $CO_2$ - Hochdruckverfahren erhaltene Extrakte aus den im folgenden aufgeführten Pflanzen:

    a) Öle bzw. Extrakte mit hohem Anteil an Alkoholen:

        Melisse, Koriander, Kardamon, Eukalyptus;

    b) Öle bzw. Extrakte mit hohem Anteil an Aldehyden:

        Eukalyptus citriodora, Zimt, Zitrone, Lemongras, Melisse, Citronella, Limette, Orange;

    c) Öle bzw. Extrakte mit hohem Anteil an Phenolen:

        Oreganum, Thymian, Rosmarin, Orange, Nelke, Fenchel, Campher, Mandarine, Anis, Cascarille, Estragon und Piment;

    d) Öle bzw. Extrakte mit hohem Anteil an Acetaten:

        Lavendel;

    e) Öle bzw. Ektrakte mit hohem Anteil an Estern:

        Senf, Zwiebel, Knoblauch;

    f) Öle bzw. Extrakte mit hohem Anteil an Terpenen:

        Pfeffer, Pomeranze, Kümmel, Dill, Zitrone, Pfefferminz, Muskatnuß.

Sofern das Prozeßhilfsmittel nur einen der genannten Aromastoffe enthält, kommen Isopropanol und Ethanol nicht zum Einsatz. Überraschenderweise hat sich gezeigt, daß eine Kombination von mindestens zwei der angegebenen Aromastoffe eine weitaus größere Wirkung aufweist, als bei einer Einzelsubstanz.

Die meisten der in der GRAS FEMA-Liste aufgeführten Aromastoffe sind nicht wasserlöslich, d.h. hydrophob. Werden sie in hauptsächlich fetthaltigen Nahrungsmitteln eingesetzt, so sind sie aufgrund ihres lipophilen Charakters direkt ohne Lösungsmittel verwendbar. Der Anteil lipophiler Nahrungsmittel ist jedoch relativ gering. Um in den meistens hydrophilen Nahrungs- oder Futtermitteln, Kosmetika oder Pharmazeutika ihre Wirkung entfalten zu können, werden sie bevorzugt in Verbindung mit einem wasserlöslichen Lösungsvermittler eingesetzt. Um dem Anspruch dieser Erfindung, gesundheitlich unbedenkliche Prozeßhilfsmittel zur Verfügung zu stellen, gerecht zu werden, werden ausschließlich für Nahrungsmittel zugelassene Lösungsvermittler-Aromastoffe, z.B. Alkohole verwendet.

Die Anwendung der Prozeßhilfsmittel erfolgt unverdünnt und/oder in wasserlöslichen Verdünnungen mit Wasser und/oder lebensmittelzulässigen Lösemitteln (z.B. Alkohole) und/oder in fettlöslichen Verdünnungen mit Pflanzen- (Fett)Ölen.

In den Prozeßhilfsmitteln können z. B. gut wasserlösliche Alkohole, bevorzugt in Konzentrationen von 0,1 bis 99 Gew-%, bezogen auf das Prozeßhilfsmittel, in Verbindung mit anderen Aromastoffen eingesetzt werden. Die Prozeßhilfsmittel können mehr als 50 % Bezylalkohol enthalten. Sie enthalten vorzugsweise weniger als 50 Gew.-% Ethanol, Isopropanol oder Benzylalkohol oder eines Gemisches dieser Stoffe. Besonders bevorzugt ist es, wenn der Anteil der genannten Alkohole bei weniger als 30 Gew.-%, insbesondere weniger als 20 Gew.-% liegt. Sofern Prozeßhilfsmittel eingesetzt werden die Bezylalkohol und wenigstens einen weiteren Aromastoff enthalten, kann der Anteil an Benzylalkohol auch bei mehr als 50 Gew.-% liegen.

Überraschenderweise haben die Prozeßhilfsmittel, die beispielsweise nur 20 Gew.-% Ethanol oder Isopropanol in Verbindung mit Aromaaldehyden und -phenolen in Konzentrationen im Promillbereich enthalten, eine sehr stark fungizide und bakterizide Wirkung; sogar Prozeßhilfsmittel, die 1 Gew.-% der genannten wasserlöslichen Alkohole in Verbindung mit weniger als 3 ‰ Aromaaldehyd und -phenol enthalten, weisen eine 70 bis 100 %-ige mikrobizide Wirkung auf.

Aus dem Voranstehenden ergibt sich, daß die erfindungsgemäßen Prozeßhilfsmittel überraschende mikrobizide Wirkungen auf das Umfeld der Produktion bzw. des Produktionsprozesses haben.

Bevorzugt ist dabei eine Verwendung der Prozeßhilfsmittel für die Produktion in Nahrungs- und Futtermitteln, Kosmetika, Pharmazeutika, Farben, Papier und/oder Zellstoffen.

In besonders bevorzugten Ausführungsformen werden die Prozeßhilfsmittel zur Haltbarkeitsverbesserung und Stabilisierung von aus der folgenden Gruppe ausgewählten Nahrungsmitteln verwendet:

Brot, Backwaren, Backmitteln, Backpulver, Puddingpulver, Getränken, diätetischen Lebensmitteln, Essenzen, Feinkost, Fisch und Fischprodukten, Kartoffeln und Produkten auf Kartoffelgrundlage, Gewürzen, Mehl, Margarine, Obst und Gemüse und Produkten auf Grundlage von Obst und Gemüse, Sauerkonserven, Stärkeprodukten, Süßwaren, Suppen, Teigwaren, Fleisch- und Fleischwaren, Milch-, Molkerei- und Käseprodukten, Geflügel und Geflügelprodukten, Ölen, Fetten und öl- oder fetthaltigen Produkten.

Das Prozeßhilfsmittel wirkt im Umfeld des für Verderbnis anfälligen Produktes, beispielsweise eines Nahrungs- oder Futtermittels, z.B. auf Maschinenteilen, die in Kontakt mit dem zu be- oder verarbeitenden Produkt stehen, oder in der Luft. Durch den direkten Kontakt mit der Oberfläche des für Verderbnis anfälligen Produktes wirken sie auch dort, d.h. sie entfalten ihre Wirkung auf der Oberfläche oder bei Eindringen in das Produkt in diesem selbst.

Der besondere Vorteil des beschriebenen Prozeßhilfsmittels ist daher, daß es einerseits zuverlässig dekontaminiert, wobei sich seine Wirksamkeit gegen Gram-positive und Gram-negative Bakterien, Pilze einschließlich Hefen und auch Viren erwiesen hat, während es andererseits für den Konsumenten des Nahrungsmittels keine Gefahr darstellt, da es für diesen vollkommen unschädlich ist und keinerlei mikrobizide, technologische Nachwirkung im Nahrungsmittel besitzt, denn die mikrobizide Wirksamkeit bezieht sich auf das Produktionsumfeld, das durch die erfindungsgemäßen Maßnahmen von kontaminierenden Mikroorganismen befreit

Das erfindungsgemäß einsetzbare Prozeßhilfsmittel kann ein Schmiermittel sein, das gleichzeitig der Schmierung, der Dekontamination der geschmierten Teile und damit indirekt der Haltbarkeitsstabilisierung der Produkte, die mit diesen Teilen in Kontakt stehen, dient.

Erfindungsgemäß kann das Prozeßhilfsmittel weiterhin ein Emulgier-, Trenn- oder Reinigungsmittel sein. Solche Mittel dienen der Emulgierung und/oder Reinigung und damit auch der Dekontamination von Flächen, Gegenständen, Maschinen, Einrichtungen, Geräten, Schneidflächen oder -vorrichtungen, Transportvorrichtungen und ähnlichem. Es kann außerdem zum Dekontaminieren und Reinigen von Nahrungsmitteln, Rohstoffen, Kosmetika, Pharmazeutika, Farben, Papier, Zellstoff, Vieh, Geflügel, Fisch und Abfällen verwendet werden.

Das erfindungsgemäß verwendbare Prozeßhilfsmittel kann darüber hinaus ein Sprühmittel sein. Ein solches Sprühmittel ermöglicht eine Feinverteilung der dekontaminierenden Wirkstoffe auf allen Maschinenteilen, Transportvorrichtungen, Schneidvorrichtungen, Arbeitsflächen usw. und kann gleichzeitig dazu führen, daß unmittelbar nach dem Schneid- bzw. Trennvorgang und/oder Verpackungs-Portionierungsvorgang verpackte Lebensmittel durch eingeschlossenes Sprühmittel in einem Klima mit dekontaminierenden und/oder haltbarkeitsstabilisierenden Eigenschaften aufbewahrt werden. Vernebel- bzw. versprühbare Ausführungsformen sind darüber hinaus wegen des vergleichsweise geringeren Bedarfs sehr kostengünstig.

Ebenso kann das Sprühmittel in und/oder auf Verpackungen, wie z.B. Tüten, Kartons oder ähnliches eingeblasen bzw. versprüht/vernebelt werden, um so das darin verpackte Produkt länger haltbar zu machen.

Die Sprühmittel dienen auch dazu, im Umfeld der Produktion (Umwelt, Kühlung, Lüftung, Frischluft) an hygienischen Schwachstellen (z.B. Kühlstrecken) vernebelt werden zu können, um somit die Keimzahl zu verringern, ohne daß das dort arbeitende Personal Schaden nimmt.

Ebenso können die Prozeßhilfsmittel zum Aufsprühen auf Nahrungsmittelflächen oder Schnittflächen eingesetzt werden, um die auf den Nahrungsmitteln befindlichen Verderbniserreger zu eliminieren oder zu reduzieren.

Ferner können diese Sprühmitel in Transporteinrichtungen, Lager und Kühlräumen und ähnlichem eingesetzt werden.

In einer weiteren Ausführungsform ist das Prozeßhilfsmittel ein Gasphasen-aktives Mittel, das der aktiven Dekontaminierung und/oder Desodorierung in der Gasphase in mehr oder weniger geschlossenen Systemen, wie Verpackungen, Abfallsystemen, Containersystemen, Transport- oder Lagerräumen und ähnlichem dient. Von der Wirkung des Gasphasenmittels profitieren sowohl das verpackte, im Container enthaltene, transportierte bzw. gelagerte Gut als auch die Luft und das jeweilige Umfeld.

Das Prozeßhilfsmittel hat sich außerdem als ein gutes Wärmeübertragungsmittel erwiesen. Mit Wärmeübertragungsmittel sind Kühl-, Heiz- und Wärmemittel gemeint, die in umlaufenden Kreislaufsystemen von flüssigen Kühl-, Heiz- und Wärmesystemen als dekontaminierende Zusätze verwendet werden können. Sie werden dabei wäßrigen oder öligen Systemen zur Verhinderung eines Wachstums von Mikroorganismen in den Flüssigkeiten zugefügt, um z. B. bei Leckagen von Kühlungen eine Kontamination zu verhindern.

In einer anderen Ausführungsform ist das Prozeßhilfsmittel ein Schneidoder Trennmittel für Schneidmesser und/oder Schneidvorrichtungen aller Art und für alle verderblichen zu schneidenden Produkte, um die Kontaminierung der Schnittstellen zu verhindern.

In der Nahrungsmittelindustrie treten oft an den Schnitt- bzw. Trennstellen von Nahrungsmitteln Kontaminationen durch Gram-negative oder Gram-positive Erreger, Schimmelpilze, Hefen und andere mögliche Verderbniserreger auf, die die Haltbarkeit der geschnittenen bzw. getrennten Produkte z.T. erheblich beeinträchtigen können und damit sowohl gesundheitliche als auch ökonomische Schäden verursachen. Die Kontaminationen werden durch Rohstoffe, Produkt/Rohstoffreste, Personal durch Maschinenteile oder betriebsbedingte Prozesse oder durch die Luft eingetragen.

Herkömmlicherweise werden daher bis heute entweder die geschnittenen bzw. getrennten oder zu schneidenden bzw. zu trennenden Nahrungsmittel pasteurisiert bzw. technisch behandelt, um sie zu dekontaminieren und damit haltbarer zu machen, oder mit Konservierungsstoffen versetzt. Wie oben bereits erwähnt, ist jedoch eine thermische Behandlung nicht in jedem Fall möglich oder zulässig und führt unter Umständen zu einer Verminderung der Qualität des Produktes.

Eine flankierende Maßnahme zur Verbesserung der Haltbarkeit von Nahrungsmitteln ist die Reinigung oder gar Desinfektion des Umfeldes mit chemischen Desinfektionsmitteln, die der Biozidregelung unterliegt. Diese Stoffe sind mehr oder weniger giftig und sollen nicht in Nahrungsmittel übertragen werden. Die chemische Desinfektion ist jedoch eine diskontinuierliche Maßnahme, die pragmatisch nur zu bestimmten Produktionszeiten an Maschinenteilen und im Umfeld eingesetzt werden kann und nach deren Durchführung ein Nachspülen mit Wasser zur Entfernung der Restsubstanzen erforderlich ist. Dementsprechend ist die direkte permanente Elimination von Verderbniserregern nicht gewährleistet.

Im Stand der Technik ist daher versucht worden, die Maschinenhygiene durch bessere Reinigungsfähigkeit oder durch Installationen zur Erzeugung bzw. Erhaltung von reiner oder keimarmer bzw. keimfreier Luft zu optimieren. Erfahrungsgemäß hat dies jedoch nicht eine erhöhte Haltbarkeit von geschnittenen bzw. getrennten Nahrungsmitteln bewirkt oder ist ökonomisch nicht mehr vertretbar oder ist praktisch nicht sicher umzusetzen.

Ein Beispiel aus der Schnittbrotindustrie zeigt, daß durch das Schneiden bzw. Trennen von Brotsorten wie Ganzteig-, Vollkorn-, Weiß-, Misch- oder Toastbrot und anschließendes Verpacken die Haltbarkeit des Schnittbrotes im Gegensatz zu Ganzbrot erheblich reduziert wird. Sie liegt je nach Brotsorte zwischen 2 und 5 Tagen. Durch die heute meistens durchgeführte anschließende thermische Behandlung (Pasteurisieren in Öfen oder Mikrowellengeräten bei einer Kerntemperatur von 60 bis 90 °C) verlängert sich die Haltbarkeit von Brot normalerweise auf 4 bis ca. 20 Tage bei Verwendung normaler dampfdurchlässiger Polyethylentütenverpackungen. Andere Folien, z.B. aus Polypropylen, die jedoch wesentlich teurer sind, können wegen ihrer geringeren Dampfdurchlässigkeit eine längere Haltbarkeit erreichen. Verpackungen mit Polyesterkunststoffen und einer eingegebenen stickstoffhaltigen Atmosphäre führen zu noch längerer Haltbarkeit. All diese Maßnahmen sind jedoch entweder sehr kostspielig oder nur für teure Spezialprodukte und -märkte einsetzbar und führen z.T. zu erheblichen Qualitätsverlusten des Schnittbrotes, z.B. durch Kondensatbildung in der Brottüte, zu weiche Brotkonsistenz oder zu frühes Austrocknen. Diese Maßnahmen lösen alle nicht die eigentlichen Ursachen der Kontamination durch den Schneid- bzw. Trennprozeß, der sowohl die im Umfeld, wie auch die in Produkt oder an der Maschine vorhandenen möglichen Verderbniserreger durch die Schneidvorrichtung, z.B. die Schneidblätter, in das Nahrungsmittel einträgt bzw. darin verteilt.

Als Schneid- bzw. Trennhilfsmittel werden üblicherweise entweder mineralische Zusammensetzungen, die in vielen Ländern nicht mehr zugelassen sind, oder pflanzliche Schneidöle eingesetzt, die oft bereits schon in sich kontaminiert, d.h. bakteriell belastet sind. Siehe z.B. G. Schuster: Investigations on mould contamination of sliced bread, Bäcker & Konditor 27(11), S. 345-347; G. Spicher: Die Quellen der direkten Kontamination des Brotes mit Schimmelpilzen; Das Schneidöl als Faktor der Schimmelkontamination; Getreide, Mehl und Brot 32(4), S. 91-94.

Für ein Schneid- bzw. Trennmittel, das eine Dekontamination der mit dem Nahrungsmittel in Kontakt stehenden Maschinenteile während des Schneidprozesses erlaubt und dadurch eine verbesserte Haltbarkeit des Schnittgutes bewirkt, besteht daher ein dringender Bedarf, der durch das erfindungsgemäße Schneid- bzw. Trennmittel befriedigt wird.

Das Schneid- oder Trennmittel ist überall einsetzbar, wo industriell gescnnitten oder getrennt wird und das Schnittgut einer Verderbnis durch Bakterien oder Pilze oder Kontamination durch Viren unterliegen kann. Dies trifft z. B. für Zellstoffe und Papier zu, besonders aber für Nahrungsoder Futtermittel.

In einer bevorzugten Ausführungsform ist das erfindungsgemäße Prozeßhilfsmittel zum Schneiden oder Trennen von Brot, Backwaren, Fisch und Fischprodukten, Kartoffeln und Produkten auf Kartoffelgrundlage, Obst und Gemüse und Produkten auf Grundlage von Obst und Gemüse, Süßwaren, Stärkeprodukten, Teigwaren, Fleisch- und Fleischwaren, Käseprodukten, Geflügel und Geflügelprodukten geeignet.

Handelt es sich bei dem Prozeßhilfsmittel um ein Schneid- bzw. Trennmittel (z.B. zum Schneiden von Brot), so kann dieses auf üblicher Pflanzenöl-Fett-Wachsbasis unter Zusatz von mikrobiziden Prozeßhilfsmitteln auf der Basis von Aromastoffen bereitgestellt werden. Das Schneid- bzw. Trennmittel (z.B. für die Anwendung in der Fleischwaren-Industrie) kann vorzugsweise erfindungsgemäß ausschließlich aus einem oder mehreren Aromastoffen bestehen.

Den Pflanzenölen, -wachsen und -fetten können auch natürliche Emulgatoren, z.B. Lecithine in einer Konzentration von 1 bis 25 Gew.-%, beigegeben werden, wie es dem Stand der Technik entspricht. Beispielhafte Emulgatoren sind Lecithine, Zitronensäuremonoglyceride, Diacetylweinsäure, N-Acetylphosphatidylethanolamin, Phosphatidylinositol, Phosphatidylserin, Phosphatidsäuren, Phosptatidylcholin. Wird das erfindungsgemäße Schneid- bzw. Trennmittel jedoch als Emulsion auf wäßriger Basis bereitgestellt, werden Pflanzenöle, Pflanzenfette und Pflanzenwachse mit ungesättigten und gesättigten $C_{16}$ - $C_{18}$-Fettsäuren, die ebenfalls eine Viskosität von ca. 10 mPas (20 °C) bis ca. 500 mPas (20 °C) haben, verwendet.

Das beispielsweise aus den oben erwähnten Fettsäuren bzw. Ölen und Emulgatoren zusammengesetzte Schneid- und Trennmittel kann nach Mischen mit Wasser im Verhältnis von 1:1 bis 1:40 als Schneid- oder Trennemulsion (-milch) angewendet werden.

In der Praxis wird das Schneid- oder Trennmittel auf mindestens die in Kontakt mit dem Schnittgut stehenden Maschinenteile aufgebracht, um diese zu dekontaminieren. Die Mittel werden erfahrungsgemäß in Dosierungen von 1-20 g/kg Nahrungsmittel eingesetzt, wobei die Dosierung von der verwendeten Schneid- bzw. Trennvorrichtung und dem Schnittgut abhängig ist.

Die Schneid- und Trennmittel werden meistens auf die Schneid- bzw. Trennvorrichtungen aufgebracht, z.B. beim Brotschneiden auf Kreistellerscheibenschneidmaschinen aufgesprüht, mit denen z.B. Schnittbrot anschließend geschnitten wird. Erfindungsgemäß werden dabei Teile der Schneidvorrichtungen, z.B. Kreistellermesser, Band-Slicer (rotierende Bandsägen), elektrische der mechanische Messer oder Messervorrichtungen, elektrische oder mechanische Sägen oder Sägevorrichtungen, elektrische oder mechanische Kettensägen oder Vorrichtungen benetzt, so daß das Schneid- bzw. Trennmittel auf dem entsprechenden Maschinenteil sowie auch auf der durch das Schneiden oder Trennen entstandenen Oberfläche dekontaminierend bzw. mikrobizid wirken kann.

Die vorteilhafte Wirkung der erfindungsgemäß einsetzbarer Schneid- und Trennmittel äußert sich in einer verlängerten Haltbarkeit des Schnittgutes, z.B. von Schnittbrot. Sie beruht nicht zuletzt darauf, daß das Schneid- und Trennmittel die Oberfläche des Schnittgutes durchdringt und auch die tieferen Schichten des geschnittenen Nahrungsmittels dekontaminiert und zwar durch die im Schneidöl enthaltenen Aromastoffe.

Die hier beschriebenen Aromastoffe wirken darüber hinaus mikrobizid in der Dampfphase, da die meisten Aromastoffe leicht verdampfen. Sie wirken daher im sogenannten Umfeld des Nahrungsmittels, z.B. in der Verpackung des Nahrungsmittels, wenn dieses nach den Schneidprozeß z.B. in eine Folienverpackung verpackt wird.

Dieser Prozeß der Dekontamination des Schnittgutes nach dem eigentlichen Schneidvorgang kann durch eine schwache thermische Nachbehandlung des

Im folgenden wird die Erfindung anhand von Beispielen näher beschrieben:

Bakteriologische Testverfahren für Additive

- Quantitativer Suspensionstest I (Keimträgerversuch)
- Quantitatives Suspensionstest II (Suspensionsversuch)
- quantitativer Suspensionstest III (Agardiffusionstest)

Mikroorganismen: Aerobe Mikroorganismen (Gesamtkeimzahl), Enterobacteriaceaen, Enterokokken, Lactobacillen, Hefen, Schimmelpilze. Bei diesen Verfahren können mit unterschiedlichen Mikroorganismen, auf unterschiedlichen Nährböden Wirkungen der Additive in Abhängigkeit von der Dosierung und Einwirkzeit ermittelt werden.

Quantitativer Suspensionstest: I - Keimträger-Versuch

Suspension je nach Testkeim:    Ringer Lösung
Tryptone Soja Bouillon
Chromcult Enterokokken Bouillon
Würze-Bouillon

Keimträger: 5 x 5 cm autoklaviertes Baumwolltuch oder Filter

Nähragar:    Gesamt-Aerobier < Plate-Count-Agar
(Caseinpepton-Glucose-Hefeextrakt Agar)
Chromocult < Enterococcus faecalis
Enterococcus faecium
Streptococcus bovis

OGYE-Selektivnährboden (Hefeextrakt-Glucose - Oxytetracyclin) Microorganismen - Schimmelpilze)

Aspergillus niger
Saccharomyces

Desoxycholat - Lactose - Agar

Microorganismen

Lactose-positive - Escherichia coli
Lactose schwach-positive - Enterobacter (cloacae)
Lactose - schwach-positive - Klebsiella (pneumoniae)
Lactose-negative - Salmonella (typhimurium u. enteritidis)
Lactose-negative - Shigella (flexneri)
Lactose-negative - Proteus (mirabilis)
Lactose-negative - Pseudomonas
Lactose-negative - Enterococcus (faecalis)

MRS-AGAR (Lactobacillus)
Lactobacillus vulgaris

Baird-Parker-Agar (mit Eigelb-Tellurit-Emulsion)

Mircoorganismen

Staphylococcus aureus
Staphylococcus epidermidis
Micrococcus (Enterococcus faecium)
Bacillus sübtilis
Hefen: Endomyces tibuliger

Cereus-Selektivagar nach Mossel (mit Eigelbemulsion)

Microorganismen

Bacillus cereus
Bacillus cereus
Bacillus subtilis
Escherichia coli
Pseudomonas aeruginosa
Proteus mirabilis
Staphylococcus aureus

Desoxycholat-Lactose-Agar

Microorganismen

Lactose-positiv-Escherichia coli
Lactose-schwach-positiv - Enterobacter (cloacae)
Lactose-schwach-positiv - Klebsiella (pneumaniae)

Lactose-negativ - Salmonella (typhimurium u. enteritidis)
Lactose-negativ - Shigella (flexneri)
Lactose-negativ - Proteus (mirabilis)
Lactose-negativ - Pseudomonas (Enterococcus faecalis)

TGE-Agar (Caseinpepton-Glucose-Fleischextrakt-Agar)

Microorganismen

Staphylococcus aureus
Streptococcus agalactiae
Enterococcus faecalis
Escherichia coli
Salmonella typhimurium
Pseudomonas aeruginosa
Bacillus cereus

Suspensionstest        Quantitativer Keimträgerversuch -
Sonstige: Spezialnährböden und Differenzierungen
Für: Clostridien, Listerien u. a.

Kontamination der Keimträger

Die Kontamination der Keimträger erfolgt nach Einlegen in eine sterile Glasschale durch Übergießen der Testkeimsuspension ($\geq 10^6$/pro ml).
Nach 1-10 min. langer Lagerung werden die Keimträger in einer mit sterilem Filterpapier ausgelegten Glasschale verteilt und im Brutschrank bei 36°C $\pm$ 1° C getrocknet.

Prüfung

Die kontaminierten und getrockneten Keimträger werden in sterile Glasschalen gelegt und mit je (gr. %/Rezep.) getränkt; 1 h gelagert und für den jeweils vorgesehenen Agar/Testkeim gelegt und im Brutschrank unter der vorgeschriebenen Temperatur bebrütet.

Nach der empfohlenen (Zeit/Bebrütung) werden die Keimträger bei 9-facher Verdünnung (je nach Testkeim) von $10^1$ bis $10^8$ verdünnt und in den jeweils vorgesehenen Agar im Plattengußverfahren eingegeben.

Berechnung:

Alle die zwischen bis 200 Kolonien aufweisen. Bestimmt mittels des gewichteten arithmetischen Mittels:

$$\overline{C} = \frac{\Sigma c}{n_1 \times 1 + n_2 \times 0,1} = x\ d$$

$\overline{C}$ = Anzahl der kolonienbildenden Einheiten je ml/g

$\Sigma c$ = Summe der Kolonien aller Petrischalen, die zur Berechnung herangezogen werden

$n_1$ = Anzahl der Petrischalen der niedrigsten Verdünnungsstufe, die zur Berechnung herangezogen werden ($n_1$ = 2 bei 2 Petrischalen)

$n_2$ = Anzahl der Petrischalen der nächsthöheren Verdünnungsstufe, die zur Berechnung herangezogen werden

$d$ = Faktor der niedrigsten ausgewerteten Verdünnungsstufe, die auf $n_1$ bezogene Verdünnungsstufe

Quantitativer Suspensionstest II       - Suspensionsversuch

a) Testkeimsuspension mit gewünschtem Testkeim, z.B. $10^6$/ml beimpften 1-60 min einwirken. Gewünschte zu prüfende Rezeptur in vorgesehene Keimsuspensionsröhrchen (unterschiedliche prozentuale Mengen) eingeben. Einwirkzeiten abwarten und in die je nach Keim entsprechenden Agarplatten eingießen oder beimpfen.

b) Testkeimsuspension vor dem Beimpfen der Testkeime (siehe a) mit der gewünschten zu prüfenden Rezeptur behandeln (siehe a). Einwirkzeiten abwarten und dann mit jeweiligen Testkeimen beimpfen und je nach Testkeim die entsprechenden Agarplatten beimpfen, oder eingießen.

Quantitativer Suspensionstest III:      AGAR-DIFFUSIONSTEST

Man gieße Nähragarplatten, die z.B. $10^4$ Microorganismen/ml enthalten.
Ein steriles Filterpapierblättchen (10 mm) wird mit der zu prüfenden Rezeptur getränkt und auf die Nähragarplatte gelegt.
Nach der Inkubation von (Zeit/Temperatur je nach Keim) wird die Bildung eines Hemmhofes als positive Reaktion abgelesen.

| Rezepturbeispiele Bakteriologische Wirksamkeit | | | | | |
|---|---|---|---|---|---|
| Milchsäure | Benzyl-Alkohol | Glycerin | Propylenglykol | Stand der Technik - Beispiele - | Rezeptur |
| $10^7$ | $10^8$ | $10^6$ | $10^9$ | 5 min. E.Z. | Gesamtkeimzahl |
| $10^7$ | $10^7$ | $10^8$ | $10^9$ | 15 min. E.Z. | |
| $10^7$ | $10^7$ | $10^8$ | $10^9$ | 60 min. E.Z. | |
| | | | | $10^8$ / ml | Kontrolle |
| $10^6$ | $10^7$ | $10^7$ | $10^8$ | 5 min. E.Z. | Enterobakterien |
| $10^6$ | $10^7$ | $10^7$ | $10^8$ | 15 min. E.Z. | |
| $10^6$ | $10^7$ | $10^7$ | $10^8$ | 60 min. E.Z. | |
| | | | | $10^8$ / ml | Kontrolle |

(fortgesetzt)

| Milchsäure | Benzyl-Alkohol | Glycerin | Propylenglykol | Stand der Technik - Beispiele - | Rezeptur |
|---|---|---|---|---|---|
| Rezepturbeispiele Bakteriologische Wirksamkeit |
| $10^7$ | $10^8$ | $10^8$ | $10^8$ | 5 min. E.Z. | Enterokokken |
| $10^7$ | $10^7$ | $10^8$ | $10^8$ | 15 min. E.Z. | |
| $10^7$ | $10^7$ | $10^8$ | $10^8$ | 60 min. E.Z. | |
| | | | | $10^8$ / ml | Kontrolle |
| $10^5$ | $10^5$ | $10^5$ | $10^5$ | 5 min. E.Z. | Lactobacillen |
| $10^4$ | $10^5$ | $10^5$ | $10^5$ | 15 min. E.Z. | |
| $10^4$ | $10^5$ | $10^5$ | $10^5$ | 60 min. E.Z. | |
| | | | | $10^5$ / ml | Kontrolle |
| $10^5$ | $10^5$ | $10^5$ | $10^5$ | 5 min. E.Z. | Hefen |
| $10^5$ | $10^4$ | $10^5$ | $10^5$ | 15 min. E.Z. | |
| $10^5$ | $10^4$ | $10^5$ | $10^5$ | 60 min. E.Z. | |
| | | | | $10^5$ / ml | Kontrolle |
| $10^5$ | $10^5$ | $10^5$ | $10^5$ | 5 min. E.Z. | Schimmelpilze |
| $10^5$ | $10^4$ | $10^5$ | $10^5$ | 15 min. E.Z. | |
| $10^5$ | $10^4$ | $10^5$ | $10^5$ | 60 min. E.Z. | |
| | | | | $10^5$ / ml | Kontrolle |

Zeichenerklärung:

(KBE) Kolonienbildende Einheiten/gr oder ml

| 3. Tannin 1 T Benzylalkohol 100 T | 2. Tannin 1 T Benzylalkohol 3T | 1 b Tannin 1 T Benzylalkohol 1 T | 1 a Tannin | Erfindung - Beispiele - 1a 1b | Rezeptur |
|---|---|---|---|---|---|
| $10^3$ | $10^2$ | $10^2$ | $10^6$ | 5 min. E.Z. | Gesamtkeimzahl |
| $10^3$ | $10^2$ | $10^2$ | $10^6$ | 15 min. E.Z. | |
| $10^3$ | $10^2$ | $10^3$ | $10^6$ | 60 min. E.Z. | |
| | | | | $10^8$ / ml | Kontrolle |
| $10^2$ | $10^1$ | $10^2$ | $10^6$ | 5 min. E.Z. | Entero-Bakterien |
| $10^2$ | $10^1$ | $10^1$ | $10^6$ | 15 min. E.Z. | |
| $10^2$ | $10^1$ | $10^1$ | $10^6$ | 60 min. E.Z. | |
| | | | | $10^8$ / ml | Kontrolle |
| $10^3$ | $10^4$ | $10^3$ | $10^7$ | 5 min. E.Z. | Entero-Kokken |
| $10^2$ | $10^3$ | $10^2$ | $10^6$ | 15 min. E.Z. | |
| $10^3$ | $10^2$ | $10^1$ | $10^6$ | 60 min. E.Z. | |
| | | | | $10^8$ / ml | Kontrolle |

(fortgesetzt)

| 3. Tannin 1 T Benzylalkohol 100 T | 2. Tannin 1 T Benzylalkohol 3T | 1 b Tannin 1 T Benzylalkohol 1 T | 1 a Tannin | Erfindung - Beispiele - 1a 1b | Rezeptur |
|---|---|---|---|---|---|
| $10^2$ | $10^1$ | $10^2$ | $10^5$ | 5 min. E.Z. | Lactobacille |
| $10^2$ | $10^1$ | $10^1$ | $10^4$ | 15 min. E.Z. | |
| $10^2$ | $10^1$ | $10^1$ | $10^4$ | 60 min. E.Z. | |
| | | | | $10^5$ / ml | Kontrolle |
| $10^2$ | $10^1$ | $10^1$ | $10^4$ | 5 min. E.Z. | Hefen |
| $10^2$ | $10^1$ | $10^1$ | $10^4$ | 15 min. E.Z. | |
| $10^1$ | $10^1$ | $10^1$ | $10^4$ | 60 min. E.Z. | |
| | | | | $10^5$ / ml | Kontrolle |
| $10^1$ | $10^1$ | $10^1$ | $10^4$ | 5 min. E.Z. | Schimmel-Pilze |
| $10^2$ | $10^1$ | $10^1$ | $10^4$ | 15 min. E.Z. | |
| $10^1$ | $10^1$ | $10^1$ | $10^4$ | 60 min. E.Z. | |
| | | | | $10^5$ / ml | Kontrolle |

| 9. Tannin 1000 T Benzylalkohol 1 T | 8. Tannin 1000 T Benzylalkohol 1 T | 7. Tannin 100 T Benzylalkohol 1 T | 6. Tannin 3 T Benzylalkohol 1 T | 5. Tannin 1 T Benzylalkohol 10000 T | 4. Tannin 1 T Benzylalkohol 1.000 T | Erfindung - Beispiele - | Rezeptur |
|---|---|---|---|---|---|---|---|
| $10^5$ | $10^4$ | $10^4$ | $10^3$ | $10^3$ | $10^3$ | 5 min. E.Z. | Gesamt-Keimzahl |
| $10^4$ | $10^5$ | $10^4$ | $10^3$ | $10^3$ | $10^3$ | 15 min. E.Z. | |
| $10^4$ | $10^4$ | $10^4$ | $10^3$ | $10^3$ | $10^4$ | 60 min. E.Z. | |
| | | | | | | $10^8$ / ml | Kontrol- |
| $10^4$ | $10^4$ | $10^3$ | $10^3$ | $10^3$ | $10^3$ | 5 min. E.Z. | Entero-Bakterien |
| $10^4$ | $10^3$ | $10^3$ | $10^2$ | $10^3$ | $10^2$ | 15 min. E.Z. | |
| $10^3$ | $10^3$ | $10^3$ | $10^2$ | $10^3$ | $10^2$ | 60 min. E.Z. | |
| | | | | | | $10^8$ / ml | Kontrolle |
| $10^4$ | $10^4$ | $10^4$ | $10^3$ | $10^3$ | $10^4$ | 5 min. E.Z. | Entero-Bakterien |
| $10^4$ | $10^4$ | $10^5$ | $10^4$ | $10^3$ | $10^3$ | 15 min. E.Z. | |
| $10^4$ | $10^4$ | $10^5$ | $10^4$ | $10^2$ | $10^3$ | 60 min. E.Z. | |
| | | | | | | $10^8$ / ml | Kontrolle |
| $10^4$ | $10^4$ | $10^4$ | $10^3$ | $10^4$ | $10^3$ | 5 min. E.Z. | Lacto-Bacille |
| $10^4$ | $10^3$ | $10^4$ | $10^4$ | $10^3$ | $10^2$ | 15 min. E.Z. | |
| $10^4$ | $10^3$ | $10^4$ | $10^3$ | $10^2$ | $10^2$ | 60 min. E.Z. | |
| | | | | | | $10^5$ / ml | Kontrolle |
| $10^3$ | $10^3$ | $10^3$ | $10^4$ | $10^3$ | $10^2$ | 5 min. E.Z. | Hefen |
| $10^3$ | $10^2$ | $10^3$ | $10^4$ | $10^2$ | $10^1$ | 15 min. E.Z. | |
| $10^3$ | $10^2$ | $10^3$ | $10^3$ | $10^2$ | $10^1$ | 60 min. E.Z. | |
| | | | | | | $10^5$ / ml | Kontrolle |
| $10^3$ | $10^4$ | $10^3$ | $10^3$ | $10^2$ | $10^1$ | 5 min. E.Z. | Schimmel-Pilze |
| $10^4$ | $10^4$ | $10^3$ | $10^3$ | $10^2$ | $10^1$ | 15 min. E.Z. | |
| $10^4$ | $10^4$ | $10^3$ | $10^3$ | $10^1$ | $10^1$ | 60 min. E.Z. | |
| | | | | | | $10^5$ / ml | Kontrolle |

| 11. Benzylalkohol 1 T Propylenglykol 10 T | 10. Benzylalkohol 1 T Propylenglykol 1 T | Erfindung - Beispiele - 1b | Rezeptur |
|---|---|---|---|
| $10^3$ | $10^3$ | 5 min. E.Z. | Gesamtkeimzahl |
| $10^4$ | $10^3$ | 15 min. E.Z. | |
| $10^4$ | $10^4$ | 60 min. E.Z. | |
| | | $10^8$ / ml | Kontrolle |
| $10^3$ | $10^3$ | 5 min. E.Z. | Enterobakterien |
| $10^4$ | $10^3$ | 15 min. E.Z. | |
| $10^3$ | $10^2$ | 60 min. E.Z. | |
| | | $10^8$ / ml | Kontrolle |
| $10^4$ | $10^4$ | 5 min. E.Z. | Enterokokken |
| $10^4$ | $10^3$ | 15 min. E.Z. | |
| $10^4$ | $10^3$ | 60 min. E.Z. | |
| | | $10^8$ / ml | Kontrolle |
| $10^4$ | $10^3$ | 5 min. E.Z. | Lactobacille |
| $10^3$ | $10^3$ | 15 min. E.Z. | |
| $10^3$ | $10^3$ | 60 min. E.Z. | |
| | | $10^5$ / ml | Kontrolle |
| $10^4$ | $10^3$ | 5 min. E.Z. | Hefen |
| $10^3$ | $10^3$ | 15 min. E.Z. | |
| $10^3$ | $10^3$ | 60 min. E.Z. | |
| | | $10^5$ / ml | Kontrolle |
| $10^3$ | $10^3$ | 5 min. E.Z. | Schimmelpilze |
| $10^3$ | $10^3$ | 15 min. E.Z. | |
| $10^3$ | $10^3$ | 60 min. E.Z. | |
| | | $10^5$ / ml | Kontrolle |

| 14. Benzylalkohol 1 T Propylenglykol 10.000 T | 13.Benzylalkohol 1 T Propylenglykol 1.000 T | 12 Benzylalkohol 1 T Propylenglykol 100 T | Erfindung - Beispiele - 1b | Rezeptur |
|---|---|---|---|---|
| $10^5$ | $10^5$ | $10^4$ | 5 min. E.Z. | Gesamtkeimzahl |
| $10^5$ | $10^4$ | $10^4$ | 15 min. E.Z. | |
| $10^5$ | $10^4$ | $10^5$ | 60 min. E.Z. | |
| | | | $10^8$ / ml | Kontrolle |
| $10^5$ | $10^5$ | $10^4$ | 5 min. E.Z. | Enterobakterien |
| $10^5$ | $10^4$ | $10^4$ | 15 min. E.Z. | |
| $10^4$ | $10^4$ | $10^4$ | 60 min. E.Z. | |
| | | | $10^8$ / ml | Kontrolle |

(fortgesetzt)

| 14. Benzylalkohol 1 T Propylenglykol 10.000 T | 13.Benzylalkohol 1 T Propylenglykol 1.000 T | 12 Benzylalkohol 1 T Propylenglykol 100 T | Erfindung - Beispiele - 1b | Rezeptur |
|---|---|---|---|---|
| $10^5$ | $10^5$ | $10^5$ | 5 min. E.Z. | Enterokokken |
| $10^5$ | $10^5$ | $10^5$ | 15 min. E.Z. | |
| $10^5$ | $10^5$ | $10^5$ | 60 min. E.Z. | |
| | | | $10^8$ / ml | Kontrolle |
| $10^4$ | $10^5$ | $10^4$ | 5 min. E.Z. | Lactobacille |
| $10^4$ | $10^4$ | $10^4$ | 15 min. E.Z. | |
| $10^4$ | $10^4$ | $10^3$ | 60 min. E.Z. | |
| | | | $10^5$ / ml | Kontrolle |
| $10^4$ | $10^4$ | $10^4$ | 5 min. E.Z. | Hefen |
| $10^4$ | $10^4$ | $10^4$ | 15 min E.Z. | |
| $10^4$ | $10^4$ | $10^4$ | 60 min. E.Z. | |
| | | | $10^5$ / ml | Kontrolle |
| $10^4$ | $10^4$ | $10^4$ | 5 min. E.Z. | Schimmelpilze |
| $10^4$ | $10^4$ | $10^4$ | 15 min. E.Z. | |
| $10^4$ | $10^3$ | $10^3$ | 60 min. E.Z. | |
| | | | $10^5$ / ml | Kontrolle |

| 16. Benzylalkohol 100 T Glycerin 1 T | 15. Benzylalkohol 10 T Glycerin 1 T | Erfindung - Beispiele - 1b 1c | Rezeptur |
|---|---|---|---|
| $10^5$ | $10^4$ | 5 min. E.Z. | Gesamtkeimzahl |
| $10^5$ | $10^4$ | 15 min. E.Z. | |
| $10^5$ | $10^4$ | 60 min. E.Z. | |
| | | $10^8$ / ml | Kontrolle |
| $10^5$ | $10^4$ | 5 min. E.Z. | Enterobakterien |
| $10^4$ | $10^4$ | 15 min. E.Z. | |
| $10^4$ | $10^4$ | 60 min. E.Z. | |
| | | $10^8$ / ml | Kontrolle |
| $10^5$ | $10^4$ | 5 min. E.Z. | Enterokokken |
| $10^5$ | $10^4$ | 15 min. E.Z. | |
| $10^4$ | $10^4$ | 60 min. E.Z. | |
| | | $10^8$ / ml | Kontrolle |
| $10^4$ | $10^4$ | 5 min. E.Z. | Lactobacille |
| $10^4$ | $10^4$ | 15 min. E.Z. | |
| $10^4$ | $10^3$ | 60 min. E.Z. | |
| | | $10^5$ / ml | Kontrolle |

(fortgesetzt)

| 16. Benzylalkohol 100 T Glycerin 1 T | 15. Benzylalkohol 10 T Glycerin 1 T | Erfindung - Beispiele - 1b 1c | Rezeptur |
|---|---|---|---|
| $10^4$ | $10^4$ | 5 min. E.Z. | Hefen |
| $10^4$ | $10^4$ | 15 min. E.Z. | |
| $10^4$ | $10^3$ | 60 min. E.Z. | |
| | | $10^5$ / ml | Kontrolle |
| $10^4$ | $10^4$ | 5 min. E.Z. | Schimmelpilze |
| $10^4$ | $10^3$ | 15 min. E.Z. | |
| $10^4$ | $10^3$ | 60 min. E.Z. | |
| | | $10^5$ / ml | Kontrolle |

| 17. Milchsäure 1 T Tannin 1 T Benzylalkohol 2 T | 18. Anisol 1 T Tannin 1 T Benzylalkohol 88 T | Erfindung - Beispiele - | Rezeptur |
|---|---|---|---|
| $10^2$ | $10^3$ | 5 min. E.Z. | Gesamtkeimzahl |
| $10^1$ | $10^3$ | 15 min. E.Z. | |
| $10^1$ | $10^3$ | 60 min. E.Z. | |
| | | $10^8$ / ml | Kontrolle |
| $10^2$ | $10^3$ | 5 min. E.Z. | Enterobakterien |
| $10^1$ | $10^3$ | 15 min. E.Z. | |
| $10^1$ | $10^2$ | 60 min. E.Z. | |
| | | $10^8$ / ml | Kontrolle |
| $10^3$ | $10^3$ | 5 min. E.Z. | Enterokokken |
| $10^3$ | $10^3$ | 15 min. E.Z. | |
| $10^2$ | $10^4$ | 60 min. E.Z. | |
| | | $10^8$ / ml | Kontrolle |
| $10^1$ | $10^2$ | 5 min. E.Z. | Lactobacille |
| $10^1$ | $10^2$ | 15 min. E.Z. | |
| $10^1$ | $10^2$ | 60 min. E.Z. | |
| | | $10^5$ / ml | Kontrolle |
| $10^1$ | $10^3$ | 5 min. E.Z. | Hefen |
| $10^1$ | $10^2$ | 15 min. E.Z. | |
| $10^1$ | $10^2$ | 60 min. E.Z. | |
| | | $10^5$ / ml | Kontrolle |
| $10^1$ | $10^2$ | 5 min. E.Z. | Schimmelpilze |
| $10^1$ | $10^1$ | 15 min. E.Z. | |
| $10^1$ | $10^1$ | 60 min. E.Z. | |
| | | $10^5$ / ml | Kontrolle |

| 20. Tannin 3 T Benzylalkohol 1 T Glycerin 96 T | 19. Tannin 3 T Benzylalkohol 1 T Wasser 96 T | Erfindung - Beispiele - 1e | Rezeptur |
|---|---|---|---|
| $10^3$ | $10^3$ | 5 min. E.Z. | Gesamtkeimzahl |
| $10^3$ | $10^3$ | 15 min. E.Z. | |
| $10^2$ | $10^3$ | 60 min. E.Z. | |
| | | $10^8$ / ml | Kontrolle |
| $10^2$ | $10^3$ | 5 min. E.Z. | Enterobakterien |
| $10^2$ | $10^2$ | 15 min. E.Z. | |
| $10^2$ | $10^2$ | 60 min. E.Z. | |
| | | $10^8$ / ml | Kontrolle |
| $10^3$ | $10^3$ | 5 min. E.Z. | Enterokokken |
| $10^3$ | $10^3$ | 15 min. E.Z. | |
| $10^2$ | $10^3$ | 60 min. E.Z. | |
| | | $10^8$ / ml | Kontrolle |
| $10^3$ | $10^3$ | 5 min. E.Z. | Lactobacille |
| $10^2$ | $10^3$ | 15 min. E.Z. | |
| $10^2$ | $10^3$ | 60 min. E.Z. | |
| | | $10^5$ / ml | Kontrolle |
| $10^4$ | $10^4$ | 5 min. E.Z. | Hefen |
| $10^3$ | $10^4$ | 15 min. E.Z. | |
| $10^3$ | $10^3$ | 60 min. E.Z. | |
| | | $10^5$ / ml | Kontrolle |
| $10^3$ | $10^3$ | 5 min. E.Z. | Schimmelpilze |
| $10^3$ | $10^3$ | 15 min. E.Z. | |
| $10^2$ | $10^3$ | 60 min. E.Z. | |
| | | $10^5$ / ml | Kontrolle |

**Patentansprüche**

1. Additiv zur Haltbarkeitsverbesserung und/oder Stabilisierung von mikrobiell verderblichen Produkten, enthaltend

   (i) ein Gemisch aus

      a) Polyphenol und
      b) Benzylalkohol und gegebenenfalls weiteren GRAS(Generally Recognized AS Safe)-Aroma-Alkoholen
      oder

   (ii) ein Gemisch aus

      c) Benzylalkohol und
      d) wenigstens einem weiteren GRAS-Aroma-Alkohol,

   wobei das Mischverhältnis der Komponenten a) zu b) und c) zu d) jeweils zwischen 1:1 bis 1:10.000 und 10.000: 1 bis 1:1 liegt, und wobei die GRAS-Aromastoffe ausgewählt sind aus Acetoin, Ethanol, 1-Propanol, 2-Propanol, Propylenglykol, Glycerin, n-Butylalkohol, 2-Methyl-1-propanol, Hexanol, L-Menthol, Octylalkohol, Zimtalkohol, 1-Phenylethanol, Heptanol, 1-Pentanol, 3-Methyl-1-butanol, 4-Methoxybenzylalkohol, Citronellol, n-Decanol, Geraniol, 3-Hexenol, Dodecanol, Linalool, Nerolidol, Nonadienol, Nonylalkohol, Rhodinol, Terpineol, Borneol, Clineol, Anisol, Cuminylalkohol, 10-Undecen-1-ol und 1-Hexadecanol.

2. Additiv nach Anspruch 1, wobei die Gemische (i) und (ii) wenigstens einen weiteren einwertigen oder mehrwertigen Alkohol mit 2 bis 10 C-Atomen, vorzugsweise 2 bis 7 C-Atomen, enthalten.

3. Additiv nach einem der Ansprüche 1 oder 2, wobei das Additiv wenigstens eine organische Säure und/oder wenigstens eines von deren physiologischen Salzen mit 1 bis 15 C-Atomen, vorzugsweise 2 bis 10 C-Atomen, enthält.

4. Additiv nach einem der Ansprüche 1 bis 3, wobei das Additiv als Komponente e) - k) Phenole, Acetate, Ester, Terpene, Acetale und/oder etherische Öle enthält.

5. Additiv nach einem der Ansprüche 1 bis 4, wobei das Additiv als Komponente I) einen Lösungsvermittler, insbesondere Glycerin, Propylenglykol, Wasser, Speiseöle und Fette, enthält.

6. Additiv nach einem der Ansprüche 1 bis 5, wobei das Verhältnis der Komponenten a) zu b) und c) zu d) zwischen 1 : 1 bis 1 : 1000 und 1000 : 1 bis 1 : 1, vorzugsweise zwischen 1 : 1 bis 1 : 100 : 1 und 100 : 1 bis 1 : 1, liegt.

7. Additiv nach Anspruch 2, wobei das Mischungsverhältnis der Komponenten a), b), c) und/oder d) zu den weiteren Alkoholen jeweils zwischen 1 : 1 und 1 : 10 000 oder 10 000 : 1 und 1 : 1, vorzugsweise zwischen 1 : 1000 bis 1 : 1 oder 1 : 1 bis 1 : 1000, liegt.

8. Additiv nach einem der Ansprüche 1 bis 7, wobei das Mischungsverhältnis der Komponenten a) oder c) zu den Komponenten e), f), g), h), i), j), k), l) jeweils zwischen 1 : 1 bis 1 : 10 000 und 10 000 : 1 bis 1 : 1, vorzugsweise zwischen 1 : 1 bis 1 : 1000 und 1000 : 1 bis 1 : 1, liegen.

9. Verfahren zur Haltbarkeitsverbesserung und/oder Stabilisierung von mikrobiell verderblichen Produkten, wobei ein Gemisch nach einem der Ansprüche 1 bis 8 dem mikrobiell verderblichen Produkt zugesetzt wird.

10. Verfahren nach Anspruch 9, wobei das Additiv in Mengen von 1 ppm bis 10 Gew.-% dem mikrobiell verderblichen Produkt zugesetzt wird.

11. Verfahren nach einem der Ansprüche 9 oder 10, wobei das Additiv in Mengen von 0,001 Gew.-% bis 0,5 Gew.-% dem mikrobiell verderblichen Produkt zugesetzt wird.

12. Verfahren nach einem der Ansprüche 8 bis 11, wobei das Additiv in Mengen von 0,002 Gew.-% bis 0,25 Gew.-% dem mikrobiell verderblichen Produkt zugesetzt wird.

13. Verfahren nach einem der Ansprüche 8 bis 12, wobei vor, nach oder während des Prozesses zur Herstellung, Verarbeitung oder Verpackung der Produkte deren Oberflächen und/oder deren Umgebung, insbesondere die Umgebungsluft und/oder die Oberflächen der unmittelbar oder mittelbar mit den Produkten in Kontakt kommenden Geräte oder sonstige Materialien mit einem oder mehreren Prozeßhilfsmitteln, beaufschlagt werden, die vorzugsweise wenigstens zwei mikrobizid wirkenden Aromastoffe enthalten.

14. Verfahren nach Anspruch 13, wobei das Prozeßhilfsmittel in.Mengen von 0,01 bis 5 g/kg, vorzugsweise 0,05 bis 2 g/kg, von Nahrungsmitteln, bei Einsatz für die Umgebungsluft in Mengen von 0,001 bis 10 g/m$^3$ Luft und auf den Oberflächen von Geräten in Mengen von 0,000001 g bis 0,1 g/m$^3$ eingesetzt wird.

15. Verwendung des Additivs nach einem der Ansprüche 1 bis 8 zur Verbesserung der Haltbarkeit und/oder Stabilisierung mikrobiell verderblicher Produkte, insbesondere von Lebensmitteln und Kosmetika.


**Claims**

1. An additive for the improvement of the keeping quality and/or stabilization of microbially perishable products, containing

(i) a mixture of

a) polyphenol and

b) benzyl alcohol and optionally other GRAS (generally recognized as safe) flavour alcohols or

(ii) a mixture of

c) benzyl alcohol and

d) at least one other GRAS flavour alcohol;

the mixing ratio of components a) to b) and c) to d) being from 1:1 to 1:10,000 and from 10,000:1 to 1:1, respectively, wherein said GRAS flavour alcohols are selected from acetoin, ethanol, 1-propanol, 2-propanol, propylene glycol, glycerol, n-butyl alcohol, 2-methyl-1-propanol, hexanol, L-menthol, octyl alcohol, cinnamyl alcohol, 1-phenylethanol, heptanol, 1-pentanol, 3-methyl-1-butanol, 4-methoxybenzyl alcohol, citronellol, n-decanol, geraniol, 3-hexenol, dodecanol, linalool, nerolidol, nonadieneol, nonyl alcohol, rhodinol, terpineol, borneol, clineol, anisole, cuminyl alcohol, 10-undecene-1-ol and 1-hexadecanol.

2. The additive according to claim 1, wherein mixtures (i) and (ii) contain at least one other monohydric or polyhydric alcohol containing from 2 to 10 carbon atoms, preferably from 2 to 7 carbon atoms.

3. The additive according to any of claims 1 or 2, wherein said additive contains at least one organic acid and/or at least one of its physiologically acceptable salts containing from 1 to 15 carbon atoms, preferably from 2 to 10 carbon atoms.

4. The additive according to any of claims 1 to 3, wherein said additive contains phenols, acetates, esters, terpenes, acetals and/or essential oils as components e) to k).

5. The additive according to any of claims 1 to 4, wherein said additive contains a solubilizer, especially glycerol, propylene glycol, water, edible oils and fats, as component l).

6. The additive according to any of claims 1 to 5, wherein the ratio of components a) to b) and c) to d) is from 1:1 to 1:1000 and from 1000:1 to 1:1, preferably from 1:1 to 1:100 and from 100:1 to 1:1.

7. The additive according to claim 2, wherein the mixing ratio of components a), b), c) and/or d) to said other alcohols is respectively from 1:1 to 1:10,000 and from 10,000:1 to 1:1, preferably from 1:1000 to 1:1 or from 1:1 to 1:1000.

8. The additive according to any of claims 1 to 7, wherein the mixing ratio of components a) or c) to components e), f), g), h), i), j), k), l) is respectively from 1:1 to 1:10,000 and from 10,000:1 to 1:1, preferably from 1:1 to 1:1000 and from 1000:1 to 1:1.

9. A method for the improvement of the keeping quality and/or stabilization of microbially perishable products, wherein a mixture as defined in any of claims 1 to 8 is added to said microbially perishable product.

10. The method according to claim 9, wherein said additive is added to said microbially perishable product in amounts of from 1 ppm to 10% by weight.

11. The method according to any of claims 9 or 10, wherein said additive is added to said microbially perishable product in amounts of from 0.001% by weight to 0.5% by weight.

12. The method according to any of claims 8 to 11, wherein said additive is added to said microbially perishable product in amounts of from 0.002% by weight to 0.25% by weight.

13. The method according to any of claims 8 to 12, wherein the surfaces of the products and/or their environment, especially the ambient air and/or the surfaces of the equipment or other materials directly or indirectly contacting the products, are treated with one or more processing aids preferably containing at least two microbicidally active flavouring agents prior to, after or during the process for the manufacturing, processing or packaging of the products.

14. The method according to claim 13, wherein said processing aid is used in amounts of from 0.01 to 5 g/kg, preferably from 0.05 to 2 g/kg, of food, or when used for the ambient air, in amounts of from 0.001 to 10 g/m$^3$ of air, or for the surfaces of equipment, in amounts of from 0.000001 g to 0.1 g/m$^3$.

15. Use of the additive according to any of claims 1 to 8 for the improvement of the keeping quality and/or stabilization of microbially perishable products, especially food products and cosmetics.

**Revendications**

1. Additif pour améliorer la conservation et/ou la stabilisation de produits sensibles à la contamination microbienne, contenant :

> (i) un mélange de :
>
>> a) polyphénol et
>> b) d'alcool benzylique et, le cas échéant, d'autres arômes à base d'alcools, généralement connus comme étant sans risque,
>
>> ou bien
>> (ii) un mélange de :
>
>> c) alcool benzylique, et
>> d) d'au moins un autre arôme à base d'alcools généralement connus comme étant sans risque,

dans lequel les proportions de mélange des composants a/b et c/d sont comprises respectivement entre 1/1 et 1/10 000, et 10 000/1 et 1/1 et dans lequel la substance formant l'arôme à base d'alcools généralement connus comme étant sans risque est choisi dans le groupe comprenant les acétoïne, éthanol, 1-propanol, 2-propanol, propylèneglycol, glycérine, alcool n-butylique, 2-méthyl-1-propanol, hexanol, L-menthol, alcool octylique, alcool cinnamique, 1-phényléthanol, heptanol, 1-pentanol, 3-méthyl-1-butanol, alcool 4-méthoxybenzylique, citronellol, n-décanol, géraniol, 3-héxenol, dodécanol, linalool, nerolidol, nonanediénol, alcool nonylique, rhodinol, terpinéol, bornéol, clinéol, anisol, alcool cuminylique, 10-undécèn-1-ol et 1-hexadécanol.

2. Additif selon la revendication 1, où les mélanges (i) et (ii) renferment un autre mono- ou polyalcool comportant 2 à 10 atomes de carbone, de préférence 2 à 7 atomes de carbone.

3. Additif selon l'une quelconque des revendications 1 ou 2, dans lequel l'additif renferme au moins un acide organique et/ou au moins un de ses sels physiologiquement acceptable comportant 1 à 15 atomes de carbone, de préférence 2 à 10 atomes de carbone.

4. Additif selon l'une quelconque des revendications 1 à 3, dans lequel l'additif renferme en tant que constituants e) à k) des phénol, acétate, ester, terpène, acétate et/ou autre huile éthérée.

5. Additif selon l'une quelconque des revendications 1 à 4, dans lequel l'additif renferme en tant que constituant 1) un agent solubilisant, en particulier la glycérine, le propylèneglycol, l'eau, l'huile de table ou la graisse.

6. Additif selon l'une quelconque des revendications 1 à 5, dans lequel les proportions des composants a/b et c/d) est compris respectivement entre 1/1 et 1/1000 et 1000/1 et 1/1, de préférence entre 1/1 et 1/100 et 1/100 et1/1.

7. Additif selon la revendication 2, dans lequel les proportions de mélange des composants a), b), c) et/ou d) par rapport aux autres alcools sont comprises respectivement entre 1/1 et 1/10 000 ou 10 000/1 et 1/1, de préférence entre 1/1000 et 1/1, ou bien 1/1 à 1/1000.

8. Additif selon l'une quelconque des revendications 1 à 5, dans lequel les proportions de mélange des composants a) ou c) par rapport aux composants e), f), g), h), i), j), k), l) sont comprises respectivement entre 1/1 et 1/10 000

et 10 000/1 et 1/1, de préférence entre 1/1 et 1/1000 et 1000/1 et 1/1.

9. Procédé pour améliorer la durée de conversation et/ou de stabilisation de produits sensibles à la contamination microbienne, dans lequel on ajoute aux produits sensibles à la contamination microbienne un mélange selon l'une quelconque des revendications 1 à 8.

10. Procédé selon la revendication 9, dans lequel l'additif est ajouté en une quantité comprise entre 1 ppm et 10 % en poids par rapport à la quantité de produit sensible à la contamination microbienne.

11. Procédé selon l'une quelconque des revendications 9 ou 10, dans lequel l'additif est ajouté en une quantité de 0,001 % en poids à 0,5 % en poids par rapport à la quantité de produit sensible à la contamination microbienne.

12. Procédé selon l'une quelconque des revendications 8 à 11, dans lequel l'additif est ajouté en une quantité de 0,002 % en poids à 0,25 % en poids par rapport à la quantité de produit sensible à la contamination microbienne.

13. Procédé selon l'une quelconque des revendications 8 à 12, dans lequel avant, après ou pendant l'opération de fabrication, de transformation ou d'emballage des produits, leur surface et/ou leur environnement, en particulier l'air environnant et/ou les surfaces des instruments en contact direct ou indirect avec le produit ou autre matériel, reçoivent une application d'un ou plusieurs adjuvants dont, de préférence, au moins deux substances consistant en un arôme ayant une action microbicide.

14. Procédé selon la revendication 13, dans lequel on utilise l'adjuvant en une quantité de 0,01 à 5 g/kg, de préférence 0,05 à 2 g/kg, des produits alimentaires, par addition à l'air environnant en des quantités comprise entre 0,001 et 10 g/m$^3$ d'air et sur la surface des instruments en une quantité comprise entre 0,000001 g à 0,1 g/m$^3$.

15. Utilisation des additifs selon l'une quelconque des revendications 1 à 8, pour améliorer la durée de conservation et/ou la stabilisation de produits sensibles à la contamination microbienne, en particulier de produits alimentaires et de produits cosmétiques.